**Europäisches Patentamt**

**European Patent Office**

**Office européen des brevets**

(19)

(11) Numéro de publication : **0 278 809 B1**

## (12) FASCICULE DE BREVET EUROPEEN

(45) Date de publication du fascicule du brevet :
**25.03.92 Bulletin 92/13**

(51) Int. Cl.$^5$ : **A61K 7/48,** A61K 35/78

(21) Numéro de dépôt : **88400098.5**

(22) Date de dépôt : **18.01.88**

(54) Nouvelles compositions cosmétiques ou dermatologiques renfermant un extrait des fruits de Silybum Marianum riche en Silymarine associé à des acides gras essentiels.

(30) Priorité : **19.01.87 FR 8700550**
**10.04.87 FR 8705089**

(43) Date de publication de la demande :
**17.08.88 Bulletin 88/33**

(45) Mention de la délivrance du brevet :
**25.03.92 Bulletin 92/13**

(84) Etats contractants désignés :
**BE CH DE ES GB GR IT LI LU NL**

(56) Documents cités :
**EP-A- 0 180 505**
**EP-A- 0 236 218**
**Handbuch der Lebensmittelschemie, J. Schor-müller, Springer Verlag 1968, Bd V, Teil 2, Seite 29-31**

(73) Titulaire : **PARFUMS ROCHAS**
**33, rue François Ier**
**F-75008 Paris (FR)**

(72) Inventeur : **Papaconstantin, Elisabeth**
**1, rue Auguste Maquet**
**F-75016 Paris (FR)**
Inventeur : **Lepage, Philippe**
**70, Boulevard Exelmans**
**F-75016 Paris (FR)**
Inventeur : **Frerejouand, Pascale**
**98, rue Jean-Jaurès**
**F-92300 Levallois-Perret (FR)**
Inventeur : **Marty, Jean-Pierre**
**17, rue Mace**
**F-78360 Montesson (FR)**

(74) Mandataire : **Ballot, Paul Denis Jacques**
**Cabinet Ballot-Schmit 7, rue le Sueur**
**F-75116 Paris (FR)**

## Description

L'invention a pour objet de nouvelles compositions cosmétiques ou dermatologiques renfermant un extrait des fruits de Silybum Marianum riche en Silymarine associé à des acides gras essentiels.

L'extrait de Silybum Marianum, appelé "Absolu d'Epine Blanche" est une substance bloquant la formation des radicaux libres. Un radical libre est un atome ou une molécule qui a acquis transitoirement un électron "célibataire". Cet électron non apparié à un autre de spin inverse, confère au radical une très grande réactivité, c'est-à-dire une grande capacité à réagir avec les molécules environnantes qui vont à leur tour devenir des radicaux libres.

Ce mécanisme permet d'expliquer la très grande toxicité de ces "molécules excitées". C'est ainsi que les radicaux libres vont s'attaquer aux éléments clés de la cellule tels que le noyau, les protéines et plus particulièrement à la membrane.

Or, les acides gras essentiels sont les constituants des phospholipides membranaires et sont particulièrement vulnérables aux radicaux libres, au niveau de leurs doubles liaisons.

Rappelons que ces doubles liaisons carbone-carbone assurent la structure et la solidité membranaire ; l'intervention des radicaux libres va introduire des cassures, des modifications d'angulation rendant la membrane moins solide, moins fluide et moins capable d'assurer la régulation des mouvements ioniques.

Ces altérations membranaires vont perturber le métabolisme de la cellule. Moins bien protégée, moins bien oxygénée et moins bien nourrie, la cellule va peu à peu perdre ses fonctions de multiplication, se scléroser puis mourir. C'est ainsi que les radicaux libres et les processus de peroxydation qu'ils engendrent, jouent un rôle significatif dans le vieillissement des cellules et consécutivement de la peau.

Il a été proposé des compositions cosmétiques à base d'extraits de fruits de Silybum Marianum dans la demande de brevet européen n° 236218. Cet extrait est associé à différents constituants dont les lipides composés d'acides gras insaturés. Par ailleurs le brevet européen n° 180505 décrit également une composition pour retarder le vieillissement de la peau consistant à appliquer de manière successive une première composition à base notamment d'insaponifiable de produits naturels comme le soja, le karité.

Il est donc particulièrement intéressant d'associer dans une composition cosmétique ou dermatologique "l'Absolu d'Epine Blanche" piégeur des radicaux libres particulièrement efficace, ayant un rôle préventif, à des acides gras essentiels ayant un rôle réparateur. L'invention a donc pour objet des compositions cosmétiques ou dermatologiques pour les soins de la peau, caractérisées en ce qu'elles renferment comme constituant principal le mélange comprenant :

– A) un extrait de fruits de Silybum Marianum riche en Silymarine appelé "Absolu d'Epine Blanche".

– B) un ou plusieurs acides gras essentiels polyinsaturés contenant 18 à 22 atomes de carbone présents uniquement :

– soit sous forme libre (1)

– soit sous forme de sels d'un métal alcalin ou d'ammonium (2)

présents sous forme d'un mélange constitué par ces 2 formes (1) et (2).

Par ailleurs, la composition peut également contenir en plus des acides gras essentiels sous forme libre ou sous forme de sels d'un métal alcalin ou d'ammonium, des acides gras essentiels sous forme de triglycérides, en mélange avec une ou les deux formes précédentes.

L'invention a plus particulièrement pour objet des compositions, caractérisées en ce que le composant B) est constitué d'acides gras essentiels polyinsaturés contenant de 18 à 22 atomes de carbone présents à la fois sous forme libre (1) et sous forme de triglycérides (3) et sous forme de sels d'un métal alcalin ou d'ammonium (2) provenant de la saponification de tout ou partie des triglycérides (3).

Par ailleurs, il est particulièrement judicieux d'ajouter au constituant A) des compositions de l'invention d'autres substances dites piégeurs de radicaux libres pour renforcer l'action de "l'Absolu d'Epine Blanche".

Ces substances sont par exemple des terpènes d'huiles essentielles oléfiniques, acycliques ou cycliques et leurs alcools dérivés et des flavonoïdes. La structure moléculaire de ces composés permet d'expliquer leur rôle de piégeurs de radicaux libres.

On peut rajouter également par exemple de la vitamine E ou alpha-tocophérol, de la vitamine C, et de la vitamine A et ses dérivés, dont les propriétés anti-oxydantes et anti-radicalaires sont largement décrites.

L'invention concerne donc tout particulièrement des compositions caractérisées en ce que le constituant A) renferme en outre des terpènes d'huiles essentielles, des flavonoïdes, de l'alpha-tocophérol.

L'invention a également pour objet des compositions caractérisées en ce qu'elles renferment comme constituant principal 0,5 à 10 % en poids du mélange comprenant :

– 10 à 30 % en poids de l'extrait de Silybum Marianum,

– 5 à 15 % en poids de terpènes d'huiles essentielles,

– 5 à 15 % en poids de flavonoïdes,

– 5 à 20 % en poids d'alpha-tocophérol,

– 25 à 40 % en poids de triglycérides d'acides gras essentiels (3),

– 10 à 25 % d'acides gras essentiels sous forme libre, de préférence l'acide gamma-linolénique (1) et sous forme de sels d'un métal alcalin ou d'ammonium (2) pouvant provenir par exemple de tout ou partie de la saponification des triglycérides (3).

Le mélange tel que défini ci-dessus contenant 25 à 40 % d'huile de bourrache est déposé sous le nom de marque de SKINAVENGER®.

Dans ces compositions, l'acide gamma-linolénique sous forme de sels d'un métal alcalin ou d'ammonium provient de préférence de la saponification de l'huile d'onagre.

La présente demande concerne aussi des compositions caractérisées en ce qu'elles renferment comme constituant principal le mélange comprenant :

– 1 à 10 % en poids d'huile d'onagre, et

– 1 à 10 % en poids du mélange lui-même constitué de :

– 5 % en extrait sec de fruits délipidés de Silybum Marianum, et

– 0,05 % à 2 % en poids d'alpha-tocophérol, dans du polyéthylène glycol.

La présente demande a plus particulièrement pour objet des compositions caractérisées en ce qu'elles renferment comme constituant principal le mélange comprenant :

– 5 % en poids d'huile d'onagre,

– 5 % en poids du mélange lui-même constitué de :

– 5 % en extrait sec de fruits délipidés de Silybum Marianum, et

– 1 % en poids d'alpha-tocophérol, dans du polyéthylène glycol ;

– 1 à 10 % en poids de filtres solaires UVA-UVB, et

– 1 à 10 % en poids d'extrait de rate.

Le mélange constitué de 5 % en extrait sec de fruits délipidés de Silybum Marianum et de 1 % en poids d'alpha-tocophérol dans du polyéthylène glycol est déposé sous le nom de marque FLAVOPHEROL®.

Les compositions telles que définies ci-dessus ont l'avantage d'associer aussi bien dans le constituant A) que dans le constituant B) certaines substances solubles dans une phase huileuse et d'autres substances solubles dans une phase aqueuse.

Ainsi, "l'Absolu d'Epine Blanche" est soluble dans l'eau, les terpènes d'huiles essentielles, les flavonoïdes et l'alpha-tocophérol étant solubles dans l'huile. De même, les triglycérides d'acides gras essentiels sont solubles dans l'huile, alors que les acides gras essentiels libres ou sous forme de sels sont solubles dans l'eau.

Dans le composant B), les triglycérides des acides gras essentiels sont les triglycérides banals de synthèse. Ces triglycérides peuvent être apportés par l'intermédiaire d'une ou plusieurs sources les renfermant à l'état naturel. De telles sources sont essentiellement les huiles végétales.

On peut citer par exemple l'huile de germe de blé, d'olive, de maïs, de tournesol, d'amande douce, d'onagre, de bourrache, de pépins de cassis. Toutes ces huiles contiennent des proportions très variables d'un ou plusieurs acides gras essentiels.

La saponification des triglycérides s'effectue par des méthodes usuelles, notamment par un hydroxyde de métal alcalin (soude ou potasse).

Cette saponification s'effectue en présence d'un anti-oxydant compte tenu de la grande facilité d'oxydation des acides gras polyinsaturés.

Les acides gras polyinsaturés présents sous forme d'un sel de métal alcalin, contenus dans le constituant B), sont de préférence des sels de sodium ou de potassium.

Il existe deux familles d'acides polyinsaturés essentiels : la famille gamma-linolénique et la famille linoléique.

Les acides gras essentiels les plus abondants au niveau cutané ont pour précurseur l'acide linoléique métabolisé suivant le schéma suivant :

```
C18 : 2 ===> C18 : 3===> C20 : 3 ========>  C20 : 4
acide          acide gamma  acide dihomo        acide
linoléique     linolénique  gamma-linolénique arachidonique
```

Tous ces acides sont donc particulièrement indispensables à la croissance normale et à l'activité physiologique des tissus.

Les huiles riches en acides gras essentiels sous forme de triglycérides cités ci-dessus sont l'huile de tournesol (57 % d'acide linoléique), l'huile de maïs (40 % acide linoléique), l'huile d'onagre (71 % acide linoléique,

EP 0 278 809 B1

9 % acide gamma-linolénique), l'huile de bourrache, l'huile de pépins de cassis.

Chez l'homme, la conversion de l'acide linoléique en acide gamma-linolénique est une étape limitante, rendue plus inopérante par de nombreuses influences extérieures. En particulier, cette étape est déficiente au cours du vieillissement.

Parmi les acides gras essentiels cités précédemment, l'acide gamma-linolénique est donc particulièrement intéressant.

L'invention a donc notamment pour objet des compositions caractérisées en ce que le composant B) est un mélange constitué d'acide gamma-linolénique sous forme libre (1), d'une huile obtenue à partir d'une source riche en acide gamma-linolénique (3) et d'acide gamma-linolénique sous forme de sels de métal alcalin ou d'ammonium (2) provenant de la saponification de (3).

L'huile d'onagre extraite des graines de plantes Oenothera Bienis ou Oenothera Bienis Lamarkiana, l'huile de bourrache et l'huile de pépins de cassis sont les trois principales huiles végétales riches en acide gamma-linolénique.

Parmi les huiles contenant de l'acide gamma-linolénique, utilisables dans les compositions de l'invention, on peut citer également l'huile obtenue par extraction de champignons siphomycètes, de spirulines, de graine d'érable, de houblon.

On peut utiliser bien entendu toute autre huile extraite d'une source naturelle d'acide gamma-linolénique.

La présente invention a tout particulièrement pour objet des compositions caractérisées en ce que le composant B) est un mélange constitué d'acide gamma-linolénique sous forme libre (1) , d'huile d'onagre (3) et de sel de métal alcalin de l'acide gamma-linolénique (2) provenant de la saponification de (3).

Les compositions selon l'invention peuvent renfermer éventuellement toutes les substances connues comme ayant des propriétés bienfaisantes sur la peau, telles que le collagène, l'élastine, l'acide hyaluronique, des humectants tels que l'urée, l'acide pyrrolidone carboxylique et ses sels, des extraits vitaminés, des parfums, des conservateurs, des colorants, des extraits de rate ou de thymus, de l'huile de jojoba, du beurre de karité, du gamma-oryzanol.

Elles peuvent contenir aussi de petites quantités de filtres ou écrans des radiations solaires à cause de l'importance des rayonnements UV sur la formation des radicaux libres de la peau. Il s'agit par exemple de filtres des radiations UVA et UVB tels que par exemple, l'hydroxy 2 - méthoxy 4 - benzophène, l'acide diméthoxy 3, 4-phényl glyoxylique sous forme de sel de sodium.

Les compositions, selon l'invention, peuvent se présenter sous toutes les formes utilisées en cosmétologie, crème ou gel en pots ou en tubes, lait, lotion en flacon de verre ou de plastique et éventuellement en flacon doseur ou flacon muni d'un compte-gouttes, ou encore en ampoules ou en bombe aérosol.

L'invention concerne donc des compositions cosmétiques ou dermatologiques, caractérisées en ce qu'elles se présentent sous forme de crème, gel, lait, pains dermatologiques, lotion pour la peau et tout particulièrement des compositions cosmétiques ou dermatologiques, caractérisées en ce que leurs excipients sont adaptés à l'application sur le visage et sur le cou.

En effet, pour chaque forme, on a recours à des excipients appropriés. Ces excipients doivent avoir toutes les qualités habituellement demandées. Ils doivent être doués d'une grande affinité pour la peau, être parfaitement bien tolérés, stables, présenter une consistance adéquate permettant une utilisation facile et agréable.

A titre d'exemples d'excipients pouvant être utilisés, on peut citer, le stéarate de glycérol, le propylène glycol, les dérivés palmitates, stéarates, la lanoline, la glycérine, l'alcool cétylique, polyol, les huiles animales, minérales, les cires, les mouillants, les épaississants, les stabilisants, les émulsionnants couramment utilisés.

Les différentes formes cosmétiques mentionnées ci-dessus sont obtenues selon les méthodes usuelles utilisées dans ce domaine.

Les composants A) et B) de la présente invention seront incorporés aux excipients et aux substances présentant des bienfaits pour la peau mentionnés ci-dessus en tenant compte de leur affinité pour les phases huiles ou eau.

Les composants B) seront incorporés normalement dès le début de l'émulsification.

Les composants A) ne seront incorporés qu'en fin d'émulsion à une température n'excédant pas 35°C.

Les exemples donnés ci-dessous illustrent l'invention:

EXEMPLE 1 :

## GEL FLUIDE

| | | |
|---|---|---|
| Polymère carboxyvinylique neutralisé | 0,25 à 0,65 | g |
| Carboxyméthyl cellulose sel de Na | 0,02 à 0,5 | g |
| Propylène glycol | 1 à 5 | g |
| Gel de polyglycérylméthacrylate | 5 à 10 | g |
| Perhydrosqualène | 1 à 5 | g |
| Gamma-Oryzanol | 0,05 à 2 | g |
| Alpha-Bisabolol | 0,05 à 1 | g |
| Skinavenger ® | 1 à 5 | g |
| Peptides de haut poids moléculaire | 1 à 5 | g |
| Acide hyaluronique | 0,01 à 0,05 | g |
| Conservateurs | q.s. | |
| Parfum | q.s. | |
| Colorants | q.s. | |
| Eau purifiée | q.s.p. 100 | g |

EXEMPLE 2 :

## EMULSION FLUIDE H/E

| | | |
|---|---|---|
| Acide stéarique | 0,5 à 2.5 | g |
| Acide gras de propylène glycol | 1 à 5 | g |
| Perhydrosqualène | 1 à 5 | g |
| Beurre de Karité ou Cacao | 1 à 5 | g |
| Polypeptides d'origine animale | 0,1 à 1 | g |
| Esters gras ramifiés | 2 à 12 | g |

| | | | | |
|---|---|---|---|---|
| Filtre UVA/UVB | 0,5 | à | 1,5 | g |
| Alpha-Bisabolol | 0,05 | à | 1 | g |
| Huile végétale | 1 | à | 5 | g |
| Gamma-Oryzanol | 0,05 | à | 0,5 | g |
| Skinavenger ® | 1 | à | 5 | g |
| Acide hyaluronique | 0,01 | à | 0,05 | g |
| Carboxyméthylcellulose neutralisée | 0,1 | à | 1 | g |
| Propylène glycol | 3 | à | 12 | g |
| Urée | 0,5 | à | 5 | g |
| Conservateurs | | | q.s. | |
| Parfum | | | q.s. | |
| Eau purifiée | | q.s.p. | 100 | g |

EXEMPLE 3 :

## CREME H/E

| | | | | |
|---|---|---|---|---|
| Emulgateur type | | | | |
|    - Ester gras P.O.E.  ) | | | | |
|    - Sucres P. O. E.    )  2 à 7 g | | | | |
|    - Phospholipides     ) | | | | |
| Huile de silicone | 0,5 | à | 5 | g |
| Cire végétale | 0,2 | à | 1 | g |
| Perhydrosqualène | 1 | à | 5 | g |
| Filtres UVA/UVB | 0,5 | à | 1,5 | g |
| Alcool cétylique | 0,2 | à | 1 | g |
| Huile végétale | 1 | à | 5 | g |
| Phytostérols | 1 | à | 2,5 | g |
| Gamma-Oryzanol | 0,05 | à | 0,5 | g |
| Triéthanolamine | 0,3 | à | 2 | g |
| Protéine d'origine animale | 1 | à | 5 | g |
| Skinavenger ® | 1 | à | 5 | g |
| Acide hyaluronique | 0,01 | à | 0,05 | g |
| Polymèrecarboxyvinylique neutralisé | 0,3 | à | 0,8 | g |
| Urée | 0,5 | à | 5 | g |
| Conservateurs | | | q.s. | |

| Parfum | q.s. |
| Eau purifiée | q.s.p. 100 g |

EXEMPLE 4 :

<u>CREME E/H</u>

| Isostéarate de glycérol et du sorbitan | 10 | à 12 | g |
|---|---|---|---|
| Perhydrosqualène | 10 | à 15 | g |
| Succinate de 2-éthyl hexyle | 10 | à 15 | g |
| Huile d'avocat | 3 | à 6 | g |
| Cire d'abeille blanche | 3 | à 5 | g |
| Gamma-Oryzanol | 0,05 | à 0,5 | g |
| Cire micro cristalline | 1 | à 3 | g |
| Skinavenger ® | 1 | à 5 | g |
| Acide hyaluronique | 0,01 | à 0,03 | g |
| Glycérine | 2 | à 5 | g |
| Sulfate de magnésium | | | |
| (Mg , SO4, 7 H2O) | | 0,7 | g |
| Conservateur | | q.s. | |
| Parfum | | q.s. | |
| Eau purifiée | | q.s.p. 100 | g |

EXEMPLE 5 :

<u>CREME DE JOUR/SOIN VISAGE</u>

| Palmitate de sorbitan | 6,0 g |
|---|---|
| Glycérides d'acides gras | 13,0 g |
| Vaseline | 4,0 g |
| Oxybenzone | 1,0 g |
| Huile d'onagre | 5,0 g |
| Sorbitol | 3,0 g |
| Polymère carboxyvinylique neutralisé | 0,5 g |
| Extrait tissulaire de rate | 3,0 g |
| Flavophérol ® | 5,0 g |
| Conservateurs | q.s. |
| Composition aromatique | q.s. |

Eau purifiée       q.s.p. 100 g

EXEMPLE 6 :

<u>CREME DE JOUR HYDRADANTE</u>

| | |
|---|---|
| Ether céthylique POE | 3,5 g |
| Stéarate de sorbitan | 3,5 g |
| Triglycérides d'acides gras | 5 g |
| Perhydrosqualène | 2 g |
| Lanoline hydrogénée | 5 g |
| Huile d'onagre | 5 g |
| Filtre solaire UVA et UVB | 2 g |
| Propylène glycol | 5 g |
| Flavophérol ® | 3 g |
| Pyrrolidone carboxylate de sodium | 2 g |
| Polymère carboxyvinylique neutralisé | 1 g |
| Conservateurs | q.s. |
| Composition aromatique | q.s. |
| Eau purifiée | q.s.p.100 g |

EXEMPLE 7 :

<u>CREME DE SOIN</u>

| | |
|---|---|
| Stéarate de glycérol et de PEG 100 | 6 g |
| Beurre de Karité | 5 g |
| Huile d'onagre | 5 g |
| Alcool cétylique | 2 g |
| Triglycérides d'acides gras | 8 g |
| Vitamine A | 0,5 g |
| Extrait biologique | 5 g |
| Flavophérol ® | 5 g |
| Glycérine | 5 g |
| Polymère carboxyvinylique neutralisé | 1 g |
| Conservateurs | q.s. |
| Composition aromatique | q.s. |
| Eau purifiée | q.s.p. 100 g |

EP 0 278 809 B1

EXEMPLE 8 :

### GEL POUR LE VISAGE

| | |
|---|---|
| AGE sous forme de liposomes | 0,5 g |
| Flavophérol (R) | 3 g |
| Pyrrolidone carboxylate de sodium | 2 g |
| Conservateur | q.s. |
| Composition aromatique | q.s. |
| Polymère carboxyvinylique neutralisé | 1,5 g |
| Propylène glycol | 5 g |
| Eau purifiée | q.s.p. 100 g |

EXEMPLE 9 :

### CREME ANTISOLAIRE

| | |
|---|---|
| Huile de vaseline | 5 g |
| Huile de silicone | 1 g |
| Ester d'acides gras | 5 g |
| Dérivé de lanoline | 8 g |
| Stéarate de glycérol et de PEG 100 | 6 g |
| Filtre solaire UVA ET UVB | 5 g |
| Huile d'onagre | 3 g |
| Sorbitol | 5 g |
| Flavophérol (R) | 3 g |
| Pigments minéraux inorganiques | 5 g |
| Polymère carboxyvinylique neutralisé | 1 g |
| Conservateurs | q.s. |
| Composition aromatique | q.s. |
| Eau purifiée | q.s.p. 100 g |

**Revendications**

1. Compositions cosmétiques ou dermatologiques pour les soins de la peau, caractérisées en ce qu'elles renferment comme constituant principal le mélange comprenant :

A) un extrait de fruits de Silybum Marianum riche en Silymarine appelé "Absolu d'Epine Blanche",

B) un ou plusieurs acides gras essentiels polyinsaturés contenant 18 à 22 atomes de carbone présents uniquement :

– soit sous forme libre (1),

– soit sous forme de sels d'un métal alcalin ou d'ammonium (2), présents sous forme d'un mélange constitué par ces deux formes (1) et (2).

2. Compositions selon la revendication 1, caractérisées en ce que les acides gras essentiels sont présents uniquement soit sous forme libre (1), soit sous forme de sels d'un métal alcalin ou d'ammonium (2), et sous forme de triglycérides (3) ou soit sous forme d'un mélange constitué par les trois formes.

9

3. Compositions selon la revendication 2, caractérisées en ce que les acides gras essentiels polyinsaturés sous forme de sels d'un métal alcalin ou d'ammonium (2) proviennent de la saponification de tout ou partie des triglycérides (3).

4. Compositions selon l'une des revendications 1, 2 ou 3, caractérisées en ce que les acides gras essentiels sont apportés à partir d'huiles végétales choisies dans le groupe comprenant les huiles de germe de blé, d'olive, de maïs, de tournesol, d'amande douce, d'onagre de bourrache, de pépins de cassis.

5. Compositions selon la revendication 1, 2, 3 ou 4, caractérisées en ce que le composant B) est un mélange constitué d'acide gamma-linolénique sous forme libre (1), d'une huile obtenue à partie d'une source riche en acide gamma-linolénique (3) et d'acide gamma-linolénique sous forme de sels de métal alcalin ou d'ammonium (2).

6. Compositions selon la revendication 5, caractérisées en ce que l'huile obtenue à partir d'une source riche en acide gamma-linolénique est choisie dans le groupe comprenant l'huile d'onagre, l'huile de bourrache, l'huile de pépins de cassis et l'huile obtenue par extraction de champignons siphomycètes, de spirulines, de graine d'érable, de houblon.

7. Compositions selon l'une des revendications précédentes, caractérisées en ce que le composant A) renferme en outre des terpènes d'huiles essentielles.

8. Compositions selon l'une des revendications précédentes, caractérisées en ce que le composant A) renferme en outre des flavonoïdes.

9. Compositions selon l'une des revendications précédentes, caractérisées en ce que le composant A) renferme en outre de l'alpha-tocophérol.

10. Compositions selon l'une des revendications précédentes, caractérisées en ce qu'elles renferment comme constituant principal 0,5 à 10 % en poids du mélange comprenant :
– 10 à 30 % en poids de l'extrait de Silybum Marianum,
– 5 à 15 % en poids de terpènes d'huiles essentielles,
– 5 à 15 % en poids de flavonoïdes,
– 5 à 20 % en poids d'alpha-tocophérol,
– 25 à 40 % en poids de triglycérides d'acides gras essentiels (3),
– 10 à 25 % d'acides gras essentiels sous forme libre et de préférence l'acide gamma- linolénique (1) et sous forme de sels d'un métal alcalin ou d'ammonium(2) pouvant provenir par exemple de tout ou partie de la saponification des triglycérides (3).

11. Compositions définies selon la revendication 10 caractérisées en ce qu'elles contiennent en tant que triglycérides d'acides gras essentiels (3), de l'huile de bourrache.

12. Compositions définies selon la revendication 11, caractérisées en ce que les acides gras essentiels sous forme de sels d'un métal alcalin ou d'ammonium (2) proviennent de la saponification de l'huile d'onagre.

13. Compositions définies selon les revendications 1 à 9, caractérisées en ce que le composant B) est un mélange constitué d'acide gamma-linolénique sous forme libre (1), d'huile d'onagre (3) et de sel de métal alcalin de l'acide gamma-linolénique (2) provenant de la saponification de (3).

14. Compositions cosmétiques ou dermatologiques pour les soins de la peau telles que définies selon une des revendications 1 à 9, et 13, caractérisées en ce qu'elles renferment comme constituant principal le mélange comprenant :
– 1 à 10 % en poids d'huile d'onagre, et
– 1 à 10 % en poids du mélange lui-même constitué :
. de 5 % (en extrait sec) de fruits délipidés de Silybum Marianum,et
. de 0,05 % à 2 % en poids d'alpha-tocophérol, dans du polyéthylène glycol.

15. Compositions telles que définies selon l'une des revendications 1 à 9 et 13, 14, caractérisées en ce qu'elles renferment comme constituant principal le mélange comprenant :
– 5 % en poids d'huile d'onagre, et
– 5 % en poids du mélange lui-même constitué :
. de 5 % (en extrait sec) de fruits délipidés de Silybum Marianum, et
. de 1 % en poids d'alpha-tocophérol, dans du polyéthylène glycol ;
– 1 % à 10 % en poids de filtres solaires UVA-UVB, et
– 1 % à 10 % en poids d'extrait de rate.

16. Compositions définies selon les revendications 1 à 15, caractérisées en ce qu'elles se présentent sous forme de crème, gel, lait, lotion pour la peau.

EP 0 278 809 B1

**Patentansprüche**

1. Kosmetische oder dermatologische Zusammensetzungen zur Pflege der Haut, dadurch gekennzeichnet, daß sie als Hauptbestandteil das Gemisch aus:

A) einem Extrakt der Früchte von Silybum Marianum, der reich an Silymarin ist und "Mariendistelextrakt" genannt wird,

B) einer oder mehreren essentiellen, mehrfach ungesättigten Fettsäuren mit 18 bis 22 Kohlenstoffatomen,
– die ausschließlich entweder in freier Form (1)
– oder in Form von Alkalimetall- oder Ammoniumsalzen (2) oder in Form eines Gemischs aus diesen beiden Formen (1) und (2) vorliegen, enthalten.

2. Zusammensetzungen nach Anspruch 1, dadurch gekennzeichnet, daß die essentiellen Fettsäuren ausschließlich entweder in freier Form (1) oder in Form von Alkalimetall- oder Ammoniumsalzen (2) und in Form von Triglyceriden (3) oder in Form eines aus diesen drei Formen gebildeten Gemischs vorliegen.

3. Zusammensetzungen nach Anspruch 2, dadurch gekennzeichnet, daß die mehrfach ungesättigten essentiellen Fettsäuren in Form von Alkalimetall- oder Ammoniumsalzen (2) aus der vollständigen oder teilweisen Verseifung von Triglyceriden (3) stammen.

4. Zusammensetzungen nach einem der Ansprüche 1, 2 oder 3, dadurch gekennzeichnet, daß die essentiellen Fettsäuren aus pflanzlichen Ölen stammen, die aus der aus Weizenkeimöl, Olivenöl, Maisöl, Sonnenblumenöl, Süßmandelöl, Nachtkerzenöl, Borretschöl und Öl aus den Kernen der schwarzen Johannisbeere bestehenden Gruppe ausgewählt sind.

5. Zusammensetzungen nach Anspruch 1, 2, 3 oder 4, dadurch gekennzeichnet, daß die Komponente B) ein Gemisch ist aus Gamma-Linolensäure in freier Form (1), einem Öl, das aus einer an Gamma-Linolensäure reichen Quelle gewonnen worden ist, (3) und Gamma-Linolensäure in Form von Alkalimetall- oder Ammoniumsalzen (2).

6. Zusammensetzungen nach Anspruch 5, dadurch gekennzeichnet, daß das Öl, das aus einer an Gamma-Linolensäure reichen Quelle gewonnen worden ist, aus der aus Nachtkerzenöl, Borretschöl, Öl aus den Kernen der schwarzen Johannisbeere und durch Extraktion von Röhrenpilzen, Spirulina-Algen, Ahornsamen oder Hopfen gewonnenem Öl bestehenden Gruppe ausgewählt ist.

7. Zusammensetzungen nach einem der vorhergehenden Ansprüche, dadurch gekennzeichnet, daß die Komponente A) zusätzlich Terpene essentieller Öle enthält.

8. Zusammensetzungen nach einem der vorhergehenden Ansprüche, dadurch gekennzeichnet, daß die Komponente A) zusätzlich Flavonoide enthält.

9. Zusammensetzungen nach einem der vorhergehenden Ansprüche, dadurch gekennzeichnet, daß die Komponente A) zusätzlich Alpha-Tocopherol enthält.

10. Zusammensetzungen nach einem der vorhergehenden Ansprüche, dadurch gekennzeichnet, daß sie als Hauptbestandteil 0,5 bis 10 Gew.-% eines Gemischs enthalten, das umfaßt:
– 10 bis 30 Gew.-% Silybum Marianum-Extrakt,
– 5 bis 15 Gew.-% Terpene essentieller Öle,
– 5 bis 15 Gew.-% Flavonoide,
– 5 bis 20 Gew.-% Alpha-Tocopherol,
– 25 bis 40 Gew.-% Triglyceride essentieller Fettsäuren (3),
– 10 bis 25 % essentielle Fettsäuren in freier Form, vorzugsweise Gamma-Linolensäure, (1) und in Form von Alkalimetall- oder Ammoniumsalzen (2), die beispielsweise aus der vollständigen oder teilweisen Verseifung von Triglyceriden (3) stammen können.

11. Zusammensetzungen nach Anspruch 10, dadurch gekennzeichnet, daß sie als Trigylceride essentieller Fettsäuren (3) Borretschöl enthalten.

12. Zusammensetzungen nach Anspruch 11, dadurch gekennzeichnet, daß die essentiellen Fettsäuren in Form von Alkalimetall- oder Ammoniumsalzen (2) aus der Verseifung von Nachtkerzenöl stammen.

13. Zusammensetzungen nach den Ansprüchen 1 bis 9, dadurch gekennzeichnet, daß die Komponente B) ein Gemisch aus Gamma-Linolensäure in freier Form (1), Nachtkerzenöl (3) und Alkalimetallsalz der Gamma-Linolensäure (2), das aus der Verseifung von (3) stammt, ist.

14. Kosmetische oder dermatalogische Zusammensetzungen zur Pflege der Haut gemäß einem der Ansprüche 1 bis 9 und 13, dadurch gekennzeichnet, daß sie als Hauptbestandteil das Gemisch aus
– 1 bis 10 Gew.-% Nachtkerzenöl und
– 1 bis 10 Gew.-% eines Gemischs enthalten, das seinerseits besteht aus:
. 5 % (als Trockenextrakt) Silybum Marianum-Früchten, deren Lipidgehalt herabgesetzt worden ist, und
. 0,05 bis 2 Gew.-% Alpha-Tocopherol in Polyethylenglykol.

15. Zusammensetzungen nach einem der Ansprüche 1 bis 9 und 13 oder 14, dadurch gekennzeichnet, daß sie als Hauptbestandteil das folgende Gemisch enthalten:
– 5 Gew.-% Nachtkerzenöl und
– 5 Gew.-% eines Gemischs, das seinerseits besteht aus
. 5 % (als Trockenextrakt) Silybum Marianum-Früchten, deren Lipidgehalt herabgesetzt worden ist, und
. 1 Gew.-% Alpha-Tocopherol in Polyethylenglykol;
– 1 bis 10 Gew.-% UVA-UVB-Sonnenfilter und
– 1 bis 10 Gew.-% Milzextrakt.

16. Zusammensetzungen nach den Ansprüchen 1 bis 15, dadurch gekennzeichnet, daß sie in Form von Creme, Gel, Milch oder Lotion für die Haut vorliegen.

## Claims

1. Cosmetic or dermatological compositions for the care of the skin, characterized in that they include as a principal constituent the mixture comprising:
A) an extract of the fruits of Silybum marianum rich in silymarine and known as "St. Mary's thistle extract",
B) one or more polyunsaturated essential fatty acids comprising 18 to 22 carbon atoms uniquely present:
– either in a free form (1), or
– in the form of salts of an alkali metal or of ammonium (2), present in the form of a mixture constituted by said two forms (1) and (2).

2. The compositions as claimed in claim 1, characterized in that the essential fatty acids are uniquely present either in a free form (1), or in the form of salts of an alkali metal or of ammonium (2), and in the form of triglycerides (3) or in the form of a mixture constituted by the three forms.

3. The compositions as claimed in claim 2, characterized in that the polyunsaturated essential fatty acids in the form of salts of an alkali metal or of ammonium (2) are derived from the complete or partial saponification of triglycerides (3).

4. The compositions as claimed in any one of claims 1, 2 and 3, characterized in that the essential fatty acids are derived from vegetable oils selected from the group comprising oils of wheat germs, of olives, of maize, of sunflowers, of sweet almonds, of evening primrose, of borage and of black currant seeds.

5. The compositions as claimed in any one of claims 1 through 4, characterized in that the component B) is a mixture made up of gamma-linolenic acid in a free form (1), of an oil obtained from a source rich in gamma-linolenic acid (3) and a gamma-linolenic acid in the form of salts of an alkali metal or of ammonium (2).

6. The compositions as claimed in claim 5, characterized in that the oil obtained from a source rich in gamma-linolenic acid is selected from the group comprising evening primrose oil, borage oil, black currant seed oil and the oil obtained by extraction from siphomycetes mushrooms, spirulina algae, maple seed and hops.

7. The compositions as claimed in any one of the preceding claims, characterized in that the component A) furthermore comprises terpenes from essential oils.

8. The compositions as claimed in any one of the preceding claims, characterized in that the component A) furthermore comprises flavonoids.

9. The compositions as claimed in any one of the preceding claims, characterized in that component A) furthermore comprises alpha-tocopherol.

10. The compositions as claimed in any one of the preceding claims, characterized in that they comprise as the principal constituent 0.5 to 10 % by weight of a mixture comprising:
– 10 to 30 % by weight of Silybum marianum extract,
– 5 to 15 % by weight of terpenes of essential oils,
– 5 to 15 % by weight of flavonoids,
– 5 to 20 % by weight of alpha-tocopherol,
– 25 to 40 % by weight of triglycerides of essential fatty acids (3),
– 10 to 25 % by weight of essential fatty acids in a free form and preferably gamma-linolenic acid (1) and in the form of salts of an alkali metal or ammonium (2) which may be derived, for instance, in all or in part from the saponification of triglycerides (3).

11. The compositions as claimed in claim 10, characterized in that they contain borage oil as said triglycerides of essential fatty acids (3).

12. The compositions as claimed in claim 11, characterized in that the essential fatty acids in the form of salts of an alkali metal or of ammonium (2) are derived from the saponification of evening primrose oil.

13. The compositions as claimed in any one of the preceding claims 1 through 9, characterized in that the component B) is a mixture consisting of gamma-linolenic acid in a free form (1), of evening primrose oil (3) and of an alkali metal salt of gamma-linolenic acid (2) derived from the saponification of (3).

14. Cosmetic or dermatological compositions for the care of the skin as claimed in any one of claims 1 through 9 and 13, characterized in that they include as the principal constituent the mixture comprising:
– 1 to 10 % by weight of evening primrose oil, and
– 1 to 10 % by weight of a mixture made up of:
. 5 % by weight (expressed as the dry extract) of delipidized fruits of Silybum marianum and
. 0.05 % by weight to 2 % by weight of alpha-tocopherol, in polyethylene glycol.

15. Compositions as claimed in any one of claims 1 through 9, 13 and 14, characterized in that they include as the principal constituent the mixture comprising:
– 5 % by weight of evening primrose oil, and
– 5 % by weight of a mixture made up of:
. 5 % by weight (expressed as the dry extract) of delipidized fruits of Silybum marianum and
. 1 % by weight of alpha-tocopherol, in polyethylene glycol,
– 1 % by weight to 10 % by weight of UVA-UVB solar filters, and
– 1 % by weight to 10 % by weight of spleen extract.

16. Compositions as claimed in any one of claims 1 through 15, characterized in that they are in the form of a creme, a gel, a milk or a lotion for the skin.